# EUROPEAN PATENT APPLICATION

(11) **EP 4 613 325 A1**
(43) Date of publication of application: **10.09.2025**
(21) Application number: 23887943.1
(22) Date of filing: 06.11.2023
(51) Int. Cl.: A61N 5/10

(54) **BORON NEUTRON CAPTURE TREATMENT SYSTEM AND WORKING METHOD THEREFOR**

(30) Priority: 07.11.2022 CN 202211385091
(71) Applicant: Neuboron Therapy System Ltd., Xiamen, Fujian 361026 (CN)
(72) Inventor: TENG, Yi-chiao, Nanjing, Jiangsu 211112 (CN); LIU, Yuanhao, Nanjing, Jiangsu 211112 (CN); CHEN, Jiang, Nanjing, Jiangsu 211112 (CN)
(74) Representative: Gunzelmann, Rainer
(86) International application number: PCT/CN2023/129864
(87) International publication number: WO 2024/099253

(57) **Abstract**

Provided are a boron neutron capture treatment system and a working method therefor. The boron neutron capture treatment system comprises an image acquiring module configured for acquiring a medical image of an irradiated body, a detecting module configured for detecting the actual concentration of blood boron in the irradiated body, a treatment planning module for generating a preset treatment plan on the basis of medical image data, a processing module for acquiring a functional relationship between the concentration of blood boron and a dose rate on the basis of the preset treatment plan and a plurality of groups of preset concentrations of blood boron, and a correction module. The correction module obtains a corrected irradiation time on the basis of the functional relationship and the actual concentration of blood boron, such that a boron-containing medicament can be prevented from being injected into a patient before treatment, thereby saving the treatment cost and simplifying the treatment process.

## Description

### TECHNICAL FIELD

The present disclosure relates to the technical field of radiotherapy and computers, and in particular to a boron neutron capture therapy (BNCT) system and a working method thereof.

### BACKGROUND

With the development of atomics, the radiotherapy such as the cobalt-60, the linear accelerator, and the electron beam has become one of major means to treat cancers. However, the conventional photon or electron therapy is restricted by physical conditions of radioactive rays. Specifically, while tumor cells are killed, a large number of normal tissues on a beam path are damaged. Due to different sensitivities of the tumor cells for the radioactive rays, the conventional radiotherapy is often undesirable to treat radioresistant malignant tumors (such as glioblastoma multiforme and melanoma).

In order to reduce radiation damages to the normal tissues surrounding the tumor, the target therapy in chemotherapy has been employed in the radiotherapy. For the highly radioresistant tumor cells, the radiotherapy with high relative biological effectiveness (RBE), including the proton therapy, the heavy particle therapy, and the neutron capture therapy, has also been developed actively. With specific aggregation of boron-containing drugs in tumor cells, and in cooperation with accurate neutron beam control, BNCT in neutron capture therapy serves as a better alternative to treat the cancers.

According to the BNCT, with a large capture cross section of the boron (¹⁰B)-containing drug for thermal neutrons, and through the ¹⁰B(n,α)⁷ Li neutron capture reaction and the nuclear fission reaction, ⁴He and ⁷Li heavy charged particles are generated. The two heavy charged particles have an average energy of about 2.33 MeV, and have characteristics of the high linear energy transfer (LET), and the short range. The alpha particle has the LET of 150 keV/µm, and the range of 8 µm. The ⁷Li heavy charged particle has the LET of 175 keV/µm, and the range of 5 µm. The total range of the two particles is approximately equivalent to a cell size, such that the radiation damage to an organism is limited to a cell level. When the boron-containing drug is aggregated to the tumor cells selectively, and an appropriate neutron source is provided, a purpose of locally killing the tumor cells on premise of no serious damage to normal tissues can be achieved.

In the BNCT, since the neutron beam for irradiating radioactive rays onto the irradiated body for treatment has the strong radioactive rays, an irradiation dose on the irradiated body is to be controlled accurately, so as to achieve the better treatment effect, and reduce the radiation damage of the radioactive rays on the irradiated body as much as possible. Hence, the accurate formulation of the treatment plan is crucial. In clinical practices, the boron-containing drugs such as the boronophenylalanine (BPA) in the BNCT are expensive in general. In order to save treatment expenses of the irradiated body and simplify the treatment process, when the treatment plan is formulated, a preset blood boron concentration is used for simulation and calculation, thereby estimating irradiation time at a prescribed dose. In actual treatment, the boron-containing drug is injected into the irradiated body continuously. Before the irradiated body enters an irradiation room, blood of the irradiated body is drawn to measure an actual blood boron concentration of the irradiated body, thereby injecting the boron-containing drug continuously in irradiation to maintain the blood boron concentration.

Different irradiated bodies have different metabolic statuses. In case of a difference between the preset blood boron concentration and the actual blood boron concentration of the irradiated body in actual irradiation, there will be an error in the treatment plan formulated according to the preset blood boron concentration, and it is laborious to perform the simulation and the calculation again according to the actual blood boron concentration of the irradiated body to obtain a new treatment plan. This prolongs total treatment time of the single irradiated body, reduces the utilization rate of the treatment system, and increases the expenses of the irradiated body. In order to improve the utilization efficiency of the treatment system, and reduce the treatment time and treatment cost of the irradiated body, the present disclosure provides a BNCT system and a working method of a treatment planning module.

### SUMMARY

In view of the above-mentioned technical problems, the present disclosure provides a BNCT system capable of reducing the treatment cost, improving the treatment efficiency and ensuring the treatment effect and a working method thereof.

According to an aspect, the present disclosure provides a BNCT system, including: an image acquisition module configured to acquire a medical image of an irradiated body; a detecting module configured to detect an actual blood boron concentration of the irradiated body; a treatment planning module configured to generate a preset treatment plan on the basis of the medical image data; a processing module configured to acquire, on the basis of the preset treatment plan and a plurality of preset blood boron concentrations, a functional relationship between each of the preset blood boron concentrations and a dose rate; and a correction module configured to acquire corrected irradiation time on the basis of the functional relationship and the actual blood boron concentration.

Further, the processing module includes a simulation portion configured to perform simulation according to the preset treatment plan and the preset blood boron concentration to obtain the dose rate corresponding to the preset blood boron concentration, and a fitting portion configured to perform fitting according to the preset blood boron concentration and the corresponding dose rate of the preset blood boron concentration to obtain the functional relationship between the blood boron concentration and the corresponding dose rate of the preset blood boron concentration.

Further, the preset blood boron concentrations are respectively 20 ppm, 25 ppm, 30 ppm, and 35 ppm.

Further, the dose rate is a total dose rate.

Further, the dose rate is a sum of a boron dose rate and a background dose rate.

Further, the dose rate is a sum of a boron dose rate, a neutron dose rate, and a photon dose rate.

Further, the dose rate is a sum of a boron dose rate, a fast neutron dose rate, an epithermal neutron dose rate, a thermal neutron dose rate, and a photon dose rate.

Further, the BNCT system further includes a control module and a neutron beam irradiation module; and the control module is configured to control the neutron beam irradiation module according to the preset treatment plan and the corrected irradiation time to perform irradiation for treatment.

Further, the BNCT system further includes a placement module configured to place the irradiated body.

According to another aspect, the present disclosure provides a working method of a BNCT system, including the following steps: generating a preset treatment plan on the basis of medical image data; acquiring, on the basis of the preset treatment plan and a plurality of different preset blood boron concentrations, a functional relationship between each of the preset blood boron concentrations and a dose rate; and acquiring corrected irradiation time on the basis of an actual blood boron concentration and the functional relationship.

Further, acquiring the functional relationship specifically includes: performing simulation on the basis of the plurality of different preset blood boron concentrations and the preset treatment plan to obtain the dose rate corresponding to each of the preset blood boron concentrations, and performing fitting on the basis of the plurality of different preset blood boron concentrations and the dose rate corresponding to each of the preset blood boron concentrations to obtain the functional relationship.

Further, acquiring the corrected irradiation time specifically includes: acquiring, on the basis of the actual blood boron concentration and the functional relationship, an estimated dose rate corresponding to the actual blood boron concentration; and correcting preset irradiation time on the basis of the estimated dose rate to obtain the corrected irradiation time.

According to the BNCT system provided by the present disclosure, the treatment planning module is configured to perform simulation according to the preset blood boron concentration to obtain a preset treatment plan. In combination with the preset treatment plan, a plurality of preset blood boron concentrations, and a dose rate corresponding to each of the preset blood boron concentrations, the processing module is configured to perform fitting to obtain a functional relationship between the preset blood boron concentration and the dose rate. The correction module is configured to perform calculation in combination with the functional relationship and the actual blood boron concentration to obtain a dose rate corresponding to the actual blood boron concentration, thereby correcting preset irradiation time to obtain corrected irradiation time. The present disclosure can omit injection of the boron-containing drug into the patient before treatment, thereby saving the treatment cost, and simplifying the treatment process.

### BRIEF DESCRIPTION OF THE DRAWINGS

FIG. 1 is a schematic view of a layout of a BNCT system according to the present disclosure;
FIG. 2 is a schematic view of a beam shaper according to the present disclosure; and
FIG. 3 is a workflow of a BNCT system according to the present disclosure.

In the figures: 1-neutron beam irradiation module, 11-neutron generation device, 111-charged particle beam generation portion, 112-target, 12-beam shaper, 121-reflector, 122-moderator, 123-thermal neutron absorber, 124-radiation shield, 125-beam outlet, 13-collimator, 3-placement module, and S-irradiated body.

### DETAILED DESCRIPTION OF THE EMBODIMENTS

To make the objectives, technical solutions, and advantages of the present disclosure clearer, the present disclosure is further described in detail below with reference to the drawings and embodiments. It should be understood that the specific embodiments described herein are merely used to explain the present disclosure, rather than to limit the present disclosure.

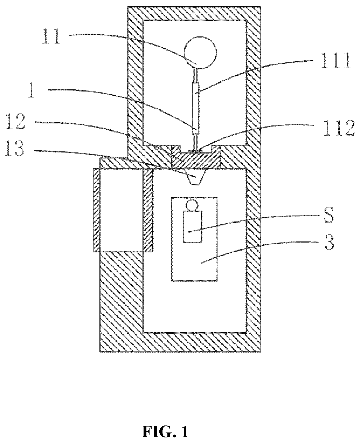

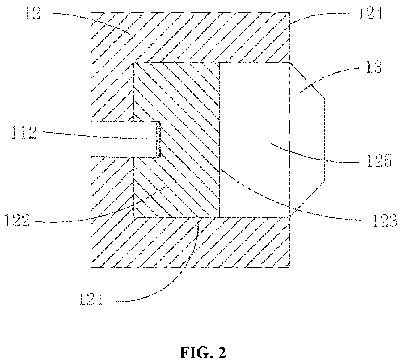

Referring to FIG. 1 and FIG. 2, a BNCT system in the embodiment includes a neutron beam irradiation module 1, an image acquisition module, a detecting module, a treatment planning module, a processing module, a correction module, a control module, and a placement module 3. Specifically, the image acquisition module is configured to acquire medical image data of an irradiated body S. The neutron beam irradiation module 1 includes a neutron generation device 11, a beam shaper 12, and a collimator 13. The neutron generation device 11 is configured to generate a neutron beam. The beam shaper 12 is configured to adjust quality of the neutron beam generated by the neutron generation device 11, thereby reducing unnecessary dose deposition. The collimator 13 is configured to gather the neutron beam, such that the neutron beam has a high targeting ability in treatment. The detecting module is configured to detect an actual blood boron concentration of the irradiated body S. The treatment planning module is configured to generate a preset treatment plan according to the medical image data of the irradiated body S and a preset blood boron concentration. The processing module is configured to acquire, on the basis of the preset treatment plan and a plurality of preset blood boron concentrations, a functional relationship between each of the preset blood boron concentrations and a dose rate. The correction module is configured to acquire corrected irradiation time in combination with the preset treatment plan, the actual blood boron concentration detected by the detecting module, and the functional relationship. The control module is configured to control the neutron beam irradiation module 1 on the basis of the preset treatment plan and the corrected irradiation time to perform irradiation for treatment. The placement module 3 is configured to place the irradiated body S.

The main principle of the BNCT is as follows: A boron (^{B-}10)-containing drug taken by or injected into the irradiated body S is gathered to the tumor cells selectively. With a large capture cross section of the boron (^{B-}10)-containing drug for thermal neutrons, and through the ¹⁰B(n,α)⁷ Li neutron capture reaction and the nuclear fission reaction, ⁴He and ⁷Li heavy charged particles are generated. The two heavy charged particles have an average energy of about 2.33 MeV, and have characteristics of the high linear energy transfer (LET), and the short range. The total range of the two particles is approximately equivalent to a cell size, such that the radiation damage to an organism is limited to a cell level, and a purpose of locally killing the tumor cells on premise of no serious damage to normal tissues can be achieved.

In the embodiment of the present disclosure, the neutron generation device 11 includes a charged particle beam generation portion 111 and a target 112. The charged particle beam generation portion 111 is configured to accelerate charged particles (such as protons and deuterons) to generate a charged particle beam such as a proton line. The charged particle beam is irradiated onto the target 112 and generates a neutron line (a neutron beam) under an action of the target 112. The target 112 is preferably a metal target 112. An appropriate nuclear reaction is selected according to characteristics such as a desired neutron yield and energy, available energies of the accelerated charged particles, a current, physical and chemical properties of the metal target 112, or the like. Nuclear reactions commonly discussed include ⁷Li(p,n)⁷Be and ⁹Be(p,n)⁹B, both of which are endothermic reactions. In the embodiment of the present disclosure, the target 112 is made of lithium. However, as is known to those skilled in the art, the target 112 may also be made of a metal material other than the lithium and beryllium, such as tantalum (Ta) or tungsten (W). The target 112 may be a circular plate, and may also be other solid shapes, and may also be made of a liquid (liquid metal). The charged particle beam generation portion may be a linear accelerator, a cyclotron, a synchrotron and a synchrocyclotron. In other implementations, the neutron generation device may be a nuclear reactor without the accelerator and the target.

No matter whether a neutron source in the BNCT comes from the nuclear reactor or the nuclear reaction between the accelerated charged particles and the target 112, a mixed radiation field is generated as a matter of fact, that is, the generated beam includes neutrons and photons having energies from low to high. As for the BNCT on deep tumors, except the epithermal neutrons, the higher the content of remaining radioactive rays, the greater the proportion causing non-selective dose deposition in the normal tissue. Therefore, the radioactive rays causing the unnecessary dose deposition should be reduced as much as possible. The beam shaper 12 can adjust the quality of the neutron beam generated by the neutron generation device 11, thereby reducing the unnecessary dose deposition. The collimator 13 is configured to gather the neutron beam, such that the neutron beam has the high targeting ability in the treatment.

The beam shaper 12 includes a moderator 121, a reflector 122, a thermal neutron absorber 123, a radiation shield 124, and a beam outlet 125. The moderator 122 can adjust energies (>40 keV) of fast neutrons from the neutron generation device 11 to an energy range (0.5 eV to 40 keV) of epithermal neutrons, and reduce a content of thermal neutrons (<0.5 eV) as much as possible. The moderator 122 is made of a material having a large action section with the fast neutrons but a small action section with the epithermal neutrons. As a preferred embodiment, the moderator 122 is made of at least one of D₂O, AlF₃, Fluental^{™}, CaF₂, Li₂CO₃, MgF₂, and Al₂O₃. The reflector 121 surrounds the moderator 122, and reflects neutrons diffused through the moderator 122 back to the neutron beam, thereby improving the utilization rate of the neutrons. The reflector is made of a material with a strong neutron reflectivity. As a preferred embodiment, the reflector 121 is made of at least one of Pb or Ni. On a transmission path of the neutron beam, the thermal neutron absorber 123 is provided behind the moderator 122, configured to absorb thermal neutrons passing through the moderator 122 to reduce the content of the thermal neutrons in the neutron beam, and made of a material having a large action section with the thermal neutrons. As a preferred embodiment, the thermal neutron absorber 123 is made of Li⁻⁶. In other embodiments, because of the Li⁻⁶ in the material of the moderator 122, the thermal neutron absorber 123 may not be provided individually, but the moderator 122 serves as the thermal neutron absorber 123. The radiation shield 124 is configured to shield neutrons and photons leaked from a portion other than the beam outlet 125. A material of the radiation shield 124 includes at least one of a photon shielding material and a neutron shielding material. As a preferred embodiment, the material of the radiation shield 124 includes lead (Pb) as the photon shielding material and polyethylene (PE) as the neutron shielding material.

The collimator 13 is provided behind the beam outlet 125. An epithermal neutron beam from the collimator 13 is irradiated onto the irradiated body S. After passing through a superficial normal tissue of the irradiated body S, the epithermal neutron beam is moderated as thermal neutrons to reach the tumor cells for treatment.

It may be understood that the beam shaper 12 may also be other structures, provided that the epithermal neutron beam can be obtained to meet the treatment requirement. In the present disclosure, the collimator 13 may also not be provided, and the beam from the beam outlet 125 of the beam shaper 12 is directly irradiated onto the irradiated body S. For ease of description, when the collimator 13 is provided, an outlet of the collimator 13 may also be understood as the beam outlet 125.

The device for acquiring the three-dimensional (3D) medical image may be an imaging device such as a calculated tomography (CT) device, a magnetic resonance imaging (MRI) device, a positron emission tomography (PET) device and an ultrasound device. In the present disclosure, preferably, the image acquisition module of the CT device is used to acquire the medical image data of the irradiated body S through CT. The medical image data of the irradiated body S includes a coordinate matrix and a CT value matrix of a medical image voxel model of a to-be-irradiated portion (a lesion, namely the tumor cells) in a medical image coordinate system.

The detecting module may be configured to detect the blood boron concentration of the irradiated body S by an inductively coupled plasma atomic emission spectroscopy (ICP-AES), a high-resolution alpha-particle autoradiography, a charged ion spectroscopy, a neutron capture camera, a nuclear magnetic resonance (NMR) and an MRI, a PET, an instantaneous gamma-ray spectrometer, etc. The device involved in the above detection methods is called the detecting module. After the boron (^{B-}10)-containing drug is taken by or injected into the irradiated body S for a period of time, the detecting module detects the blood boron concentration of the irradiated body S.

The placement module 3 includes a placement table configured to support the irradiated body S and a drive portion configured to drive the placement table to move to a preset position.

There are the following main contributing doses in the BNCT:
1) Boron dose DB: The boron dose comes from high-LET alpha particles and high-LET ⁷Li particles generated by a ¹⁰B(n,α)⁷Li neutron capture reaction between the boron-containing drug in the tissue and tumor and the neutrons.
2) Neutron dose: The neutron dose includes a fast neutron dose Df, an epithermal neutron dose Depi, and a thermal neutron dose Dth. The fast neutron dose is generated by an interaction between neutrons with energies greater than 10 keV and the elements in the tissue. The epithermal neutron dose Depi is generated by an interaction between neutrons with energies between 0.5 eV to 10 keV and the elements in the tissue. The thermal neutron dose is generated by an interaction between neutrons with energies less than 0.5 eV and the elements in the tissue.
3) Photon dose Dγ: The photon dose includes photons induced by an interaction between the neutrons and the shielding structure, and a capture reaction between the neutrons and the tissue. The latter is mainly the photons at 2.22 MeV generated in a ¹H(n, γ)²H reaction of the thermal neutrons.

The dose rate refers to a dose received in unit time. The dose rate includes a boron dose rate, a fast neutron dose rate, an epithermal neutron dose rate, a thermal neutron dose rate, and a photon dose rate. The neutron dose rate and the photon dose rate are called a background dose rate. The total dose rate is a sum of the boron dose rate and the background dose rate.

The processing module includes a simulation portion configured to perform simulation according to the preset treatment plan and the plurality of preset blood boron concentrations to obtain the dose rate corresponding to each of the plurality of different preset blood boron concentrations, and a fitting portion configured to perform fitting according to the plurality of preset blood boron concentrations and the corresponding dose rate of each of the preset blood boron concentrations in the simulation portion to obtain the functional relationship between the blood boron concentration and the corresponding dose rate of the preset blood boron concentration.

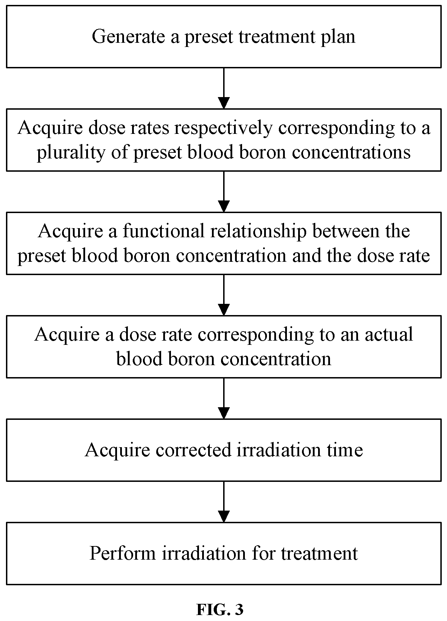

Referring to FIG. 3, specifically, the present disclosure provides a working method of a BNCT system, including the following steps:

A preset treatment plan is generated:

A treatment planning module performs dose simulation and calculation according to 3D medical image data of an irradiated body S and a preset blood boron concentration, and generates the preset treatment plan including a beam parameter, an irradiation position, an irradiation direction, a prescribed dose, and preset irradiation time, etc.

Specifically, a prescribed dose is set by a medical worker in combination with a physical characteristic parameter of the irradiated body, the medical image data of the irradiated body, and experience of the medical worker. The prescribed dose is a target irradiation dose actually received by the irradiated body. In combination with the medical image data and the preset blood boron concentration, the treatment planning module performs simulation through a Monte Carlo simulation program to calculate a dose distribution of the irradiated body S in the irradiation, and generates the preset treatment plan.

A functional relationship between a plurality of preset blood boron concentrations and dose rates respectively corresponding to the preset blood boron concentrations is acquired:

A simulation portion of a processing module acquires a plurality of different preset blood boron concentrations. While keeping the beam parameter, the irradiation position, the irradiation direction, and the prescribed dose in the preset treatment plan unchanged, the simulation portion performs simulation on the preset blood boron concentrations sequentially to obtain the dose rates respectively corresponding to the plurality of different preset blood boron concentrations. A fitting portion performs function fitting according to the plurality of preset blood boron concentrations and the dose rates respectively corresponding to the plurality of preset blood boron concentrations to generate a calculation equation for characterizing the functional relationship between the preset blood boron concentration and the dose rate.

In other embodiments, while keeping the beam parameter, the irradiation position, the irradiation direction, and the preset irradiation time in the preset treatment plan unchanged, the simulation portion performs simulation on the preset blood boron concentrations sequentially to obtain the dose rates respectively corresponding to the plurality of different preset blood boron concentrations.

The preset blood boron concentrations are set according to experience in clinic trials. Generally, when an injection dose of the boron-containing drug is 400 mg/kg, the blood boron concentrations of the irradiated body S in most cases are in a range from about 20 ppm to 35 ppm. The closer the preset blood boron concentrations are to an actual blood boron concentration, the more accurate the formulated treatment plan. When the preset blood boron concentrations are selected in the range from 20 ppm to 35 ppm, the formulated treatment plan is accurate. In an embodiment of the present disclosure, the preset blood boron concentrations are respectively set as 20 ppm, 25 ppm, 30 ppm, and 35 ppm.

In other embodiments, a number of the preset blood boron concentrations and specific values of the preset blood boron concentrations may be adjusted according to an actual condition of the irradiated body S.

Preset irradiation time is corrected:
In combination with the actual blood boron concentration actually measured by a detecting module and the calculation equation for characterizing the functional relationship between the blood boron concentration and the dose rate, a correction module obtains an estimated dose rate corresponding to the actual blood boron concentration, and corrects preset irradiation time on the basis of the estimated dose rate to obtain corrected irradiation time.

An irradiation dose rate changes with the blood boron concentration. When the prescribed dose is unchanged, and the blood boron concentration changes, the irradiation time is to be adjusted to ensure that the irradiation dose actually received by the irradiated body is the same as the prescribed dose to guarantee the treatment effect.

Irradiation is performed for treatment.

A control module invokes a preset treatment plan corresponding to a present irradiated body S from the treatment planning module, controls a placement module 3 to move the irradiated body S to the irradiation position, and controls a neutron beam irradiation module 1 according to the corrected irradiation time to generate a neutron beam to irradiate the irradiated body S.

Upon completion of the irradiation, an actual blood boron concentration of the irradiated body S is detected. According to the actual blood boron concentration before the irradiation and the actual blood boron concentration after the irradiation, an average blood boron concentration is calculated. In combination with the actual irradiation time, the irradiation dose actually received by the irradiated body S is calculated, thereby evaluating the treatment effect.

Hereinafter, through a difference between the estimated dose rate calculated by the treatment planning module in the present disclosure and the actual dose rate calculated by the Monte Carlo simulation, the accuracy of the estimated dose rate obtained by the treatment planning module in the present disclosure is verified.

In Embodiment 1 of the present disclosure, the dose rate is a total dose rate. That is, the calculation equation characterizes the functional relationship between the blood boron concentration and the total dose rate. The estimated total dose rate calculated through the calculation equation obtained by the processing module in the present disclosure is compared with the actual total dose rate calculated through the Monte Carlo simulation. In an embodiment, the preset blood boron concentrations are respectively set as 20 ppm, 25 ppm, 30 ppm, and 35 ppm. According to these preset blood boron concentrations and the total dose rates respectively corresponding the preset blood boron concentrations, fitting is performed to obtain the calculation equation. The actual blood boron concentrations detected by the detecting module are respectively 17.5 ppm, 22.5 ppm, 27.5 ppm, 32.5 ppm, and 37.5 ppm. The actual blood boron concentration is brought into the calculation equation to obtain the estimated total dose rate. According to the Monte Carlo simulation, the actual total dose rate is calculated in combination with the actual blood boron concentration and the medical image data. The specific calculated results are listed in Table 1 to Table 4.

**Table 1: Estimated total dose rate, actual total dose rate, and difference between the estimated total dose rate and the actual total dose rate when the target tissue is a brain**

| Calculation equation | y = -9E-05x2 + 0.0218x + 0.3843, R² = 0.9997 | | | | |
|---|---|---|---|---|---|
| Actual blood boron concentration (ppm) | 17.5 | 22.5 | 27.5 | 32.5 | 37.5 |
| Actual total dose rate (cGy-Eq/s) | 0.7374 | 0.8301 | 0.9175 | 0.9886 | 1.0705 |
| Estimated total dose rate (cGy-Eq/s) | 0.7382 | 0.8292 | 0.9157 | 0.9977 | 1.0752 |
| Difference between the estimated total dose rate and the actual total dose rate | 0.111% | -0.106% | -0.197% | 0.924% | 0.447% |

**Table 2: Estimated total dose rate, actual total dose rate, and difference between the estimated total dose rate and the actual total dose rate when the target tissue is a mucous membrane**

| Calculation equation | y = -9E-05x² + 0.0313x + 0.5236, R² = 1 | | | | |
|---|---|---|---|---|---|
| Actual blood boron concentration (ppm) | 17.5 | 22.5 | 27.5 | 32.5 | 37.5 |
| Actual total dose rate (cGy-Eq/s) | 1.0425 | 1.1853 | 1.3275 | 1.4495 | 1.5611 |
| Estimated total dose rate (cGy-Eq/s) | 1.0438 | 1.1823 | 1.3163 | 1.4458 | 1.5708 |
| Difference between the estimated total dose rate and the actual total dose rate | 0.125% | -0.253% | -0.847% | -0.259% | 0.619% |

**Table 3: Estimated total dose rate, actual total dose rate, and difference between the estimated total dose rate and the actual total dose rate when the target tissue is a tumor Dmax**

| Calculation equation | y = -0.0029x² + 0.3511x -0.4019, R² = 1 | | | | |
|---|---|---|---|---|---|
| Actual blood boron concentration (ppm) | 17.5 | 22.5 | 27.5 | 32.5 | 37.5 |
| Actual total dose rate (cGy-Eq/s) | 4.7942 | 5.9767 | 7.0514 | 7.9536 | 8.6793 |
| Estimated total dose rate (cGy-Eq/s) | 4.8542 | 6.0297 | 7.0602 | 7.9457 | 8.6862 |
| Difference between the estimated total dose rate and the actual total dose rate | 1.252% | 0.887% | 0.125% | -0.099% | 0.080% |

**Table 4: Estimated total dose rate, actual total dose rate, and difference between the estimated total dose rate and the actual total dose rate when the target tissue is a tumor D₉₅**

| Calculation equation | y = -0.0005x² + 0.1886x + 1.518, R² = 0.9997 | | | | |
|---|---|---|---|---|---|
| Actual blood boron concentration (ppm) | 17.5 | 22.5 | 27.5 | 32.5 | 37.5 |
| Actual total dose rate (cGy-Eq/s) | 4.5864 | 5.5365 | 6.3128 | 7.1213 | 7.8731 |
| Estimated total dose rate (cGy-Eq/s) | 4.6654 | 5.5084 | 6.3264 | 7.1194 | 7.8874 |
| Difference between the estimated total dose rate and the actual total dose rate | 1.721% | -0.508% | 0.215% | -0.027% | 0.181% |

Through comparison on data from Table 1 to Table 4, the following conclusions can be drawn intuitively: The difference between the estimated total dose rate calculated through the calculation equation obtained by the processing module in the present disclosure and the actual total dose rate calculated through the Monte Carlo simulation is within 2%, and within 1% in most cases. During radiotherapy, the positioning requirement in treatment can be met, provided that the error of the dose received by the irradiated body S is not greater than 5%. Therefore, the estimated total dose rate calculated through the calculation equation obtained by the processing module in the present disclosure has the high accuracy, and all calculated results are within an acceptable error range.

In Embodiment 2 of the present disclosure, the dose rate is the sum of the boron dose rate and the background dose rate. That is, the calculation equation characterizes the functional relationship between the blood boron concentration and the boron dose rate, and the functional relationship between the blood boron concentration and the background dose rate. Hereinafter, the estimated boron dose rate and the estimated background dose rate calculated through the calculation equation obtained by the processing module in the present disclosure are compared with the actual boron dose rate and the actual background dose rate calculated through the Monte Carlo simulation. In the embodiment, the preset blood boron concentrations are respectively set as 20 ppm, 25 ppm, 30 ppm, and 35 ppm. According to these preset blood boron concentrations, the boron dose rates respectively corresponding the preset blood boron concentrations, and the background dose rate, fitting is performed to obtain the first calculation equation for characterizing the preset blood boron concentration and the boron dose rate, and a second calculation equation for characterizing the preset blood boron concentration and the background dose rate. The actual blood boron concentration is 17.5 ppm, 22.5 ppm, 27.5 ppm, 32.5 ppm, and 37.5 ppm. The specific calculated results are listed in Table 5. The actual total dose rate is a sum of the actual boron dose rate calculated through the Monte Carlo simulation and the actual background dose rate. The estimated total dose rate is a sum of the estimated boron dose rate calculated through the calculation equation obtained by the processing module in the present disclosure and the estimated background dose rate.

**Table 5: Estimated total dose rate, actual total dose rate, and difference between the estimated total dose rate and the actual total dose rate when the target tissue is a brain**

| First calculation equation | y = -4E-05x² + 0.0193x + 0.0337, R² = 1 | | | | |
|---|---|---|---|---|---|
| Second calculation equation | y = -6E-05x2 + 0.0025x + 0.3507, R² = 0.8044 | | | | |
| Actual blood boron concentration (ppm) | 17.5 | 22.5 | 27.5 | 32.5 | 37.5 |
| Actual boron dose rate (cGy-Eq/s) | 0.3574 | 0.4501 | 0.5366 | 0.6196 | 0.7013 |
| Actual background dose rate (cGy-Eq/s) | 0.3800 | 0.3800 | 0.3810 | 0.3690 | 0.3692 |
| Actual total dose rate (cGy-Eq/s) | 0.7374 | 0.8301 | 0.9175 | 0.9886 | 1.0705 |
| Estimated boron dose rate (cGy-Eq/s) | 0.3592 | 0.4477 | 0.5342 | 0.6187 | 0.7012 |
| Estimated background dose rate (cGy-Eq/s) | 0.3761 | 0.3766 | 0.3741 | 0.3686 | 0.3601 |
| Estimated total dose rate (cGy-Eq/s) | 0.7353 | 0.8243 | 0.9083 | 0.9873 | 1.0613 |
| Difference between the estimated total dose rate and the actual total dose rate | -0.291 % | -0.704% | -1.010% | -0.134% | -0.857% |

Through comparison on data in Table 5, the following conclusions can be drawn intuitively: The difference between the estimated total dose rate calculated through the calculation equation obtained by the processing module in the present disclosure and the actual total dose rate calculated through the Monte Carlo simulation is within 2%, and within 1% in most cases. During radiotherapy, the positioning requirement in treatment can be met, provided that the error of the dose received by the irradiated body S is not greater than 5%. Therefore, the estimated total dose rate calculated through the calculation equation obtained by the processing module in the present disclosure has the high accuracy, and all calculated results are within an acceptable error range.

From Table 1 to Table 5, R² is a coefficient of determination for the functional relationship. The closer the coefficient of determination is to 1, the higher the conformity between the data and the functional relationship.

As can be seen from the above, for different tissues, the difference between the estimated total dose rate obtained by the processing module of the present disclosure and the actual total dose rate calculated through the Monte Carlo simulation is within the acceptable error range, which can ensure the accuracy of the treatment plan.

According to the BNCT system provided by the present disclosure, the treatment planning module is configured to perform simulation according to the preset blood boron concentration to obtain a preset treatment plan. In combination with the preset treatment plan, a plurality of preset blood boron concentrations, and a dose rate corresponding to each of the preset blood boron concentrations, the processing module is configured to perform fitting to obtain a functional relationship between the preset blood boron concentration and the dose rate. The correction module is configured to perform calculation in combination with the functional relationship and the actual blood boron concentration to obtain a dose rate corresponding to the actual blood boron concentration, thereby correcting preset irradiation time to obtain corrected irradiation time. The present disclosure can omit injection of the boron-containing drug into the patient before treatment, thereby saving the treatment cost, and simplifying the treatment process. The present disclosure can quickly calculate the corresponding dose rate according to the actual blood boron concentration, and corrects the irradiation time. This simplifies the process for acquiring the corresponding irradiation time according to the actual blood boron concentration, shortens the treatment time, improves the utilization rate of the device, and reduces the irradiation error due to the change of the blood boron concentration in actual irradiation.

Further, in other embodiments, the dose rate may be a sum of a boron dose rate, a neutron dose, and a photon dose rate, and may also be a sum of a boron dose rate, a fast neutron dose rate, an epithermal neutron dose rate, a thermal neutron dose rate, and a photon dose rate.

All calculation equations are quadratic functional relationships in the embodiment of the present disclosure, and may also be other functional relationships in other embodiments.

In other embodiments, the preset blood boron concentration may be any value, and two adjacent blood boron concentrations are not spaced by 10 ppm. In addition, there may be another number of blood boron concentrations. In principle, the greater the number of blood boron concentrations, the closer the coefficient of determination for the fitting function between the dose rate and the blood boron concentration is to 1, namely the higher the conformity between the data and the functional relationship. However, in view of time cost caused by the simulation, there are no more than 20 preset blood boron concentrations in general.

It should be understood that although the steps in the flowcharts in the above embodiments are shown in sequence as indicated by the arrows, these steps are not necessarily performed in sequence as indicated by the arrows. The execution order of these steps is not strictly limited, and these steps may be executed in other orders, unless clearly described otherwise. Moreover, at least some of the steps in the flowcharts in the above embodiments may include a plurality of steps or stages. The steps or stages are unnecessarily executed at the same time, but may be executed at different times. The execution order of the steps or stages is unnecessarily carried out sequentially, but may be executed alternately with other steps or at least some of the steps or stages of other steps.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

The above embodiments are merely illustrative of several implementations of the present disclosure, and the description thereof is more specific and detailed, but is not to be construed as a limitation to the patentable scope of the present disclosure. It should be noted that, for a person of ordinary skill in the art, several variations and improvements can be made without departing from the concept of the present application, all of which fall within the protection scope of the present application. Therefore, the protection scope of the present disclosure should be subject to the protection scope defined by the claims.

## Claims

1. A boron neutron capture therapy (BNCT) system, comprising:
an image acquisition module configured to acquire a medical image of an irradiated body;
a detecting module configured to detect an actual blood boron concentration of the irradiated body;
a treatment planning module configured to generate a preset treatment plan on the basis of the medical image data;
a processing module configured to acquire, on the basis of the preset treatment plan and a plurality of preset blood boron concentrations, a functional relationship between each of the preset blood boron concentrations and a dose rate; and
a correction module configured to acquire corrected irradiation time on the basis of the functional relationship and the actual blood boron concentration.

2. The BNCT system according to claim 1, wherein the processing module comprises a simulation portion configured to perform simulation according to the preset treatment plan and the preset blood boron concentration to obtain the dose rate corresponding to the preset blood boron concentration, and a fitting portion configured to perform fitting according to the preset blood boron concentration and the corresponding dose rate of the preset blood boron concentration to obtain the functional relationship between the blood boron concentration and the corresponding dose rate of the preset blood boron concentration.

3. The BNCT system according to claim 1, wherein the preset blood boron concentrations are respectively 20 ppm, 25 ppm, 30 ppm, and 35 ppm.

4. The BNCT system according to claim 1, wherein the dose rate is a total dose rate.

5. The BNCT system according to claim 1, wherein the dose rate is a sum of a boron dose rate and a background dose rate.

6. The BNCT system according to claim 1, wherein the dose rate is a sum of a boron dose rate, a neutron dose rate, and a photon dose rate.

7. The BNCT system according to claim 1, wherein the dose rate is a sum of a boron dose rate, a fast neutron dose rate, an epithermal neutron dose rate, a thermal neutron dose rate, and a photon dose rate.

8. The BNCT system according to claim 1, further comprising a control module and a neutron beam irradiation module, wherein the control module is configured to control the neutron beam irradiation module according to the preset treatment plan and the corrected irradiation time to perform irradiation for treatment.

9. The BNCT system according to claim 1, further comprising a placement module configured to place the irradiated body.

10. A working method of a boron neutron capture therapy (BNCT) system, comprising the following steps:
generating a preset treatment plan on the basis of medical image data;
acquiring, on the basis of the preset treatment plan and a plurality of different preset blood boron concentrations, a functional relationship between each of the preset blood boron concentrations and a dose rate; and
acquiring corrected irradiation time on the basis of an actual blood boron concentration and the functional relationship.

11. The working method according to claim 10, wherein acquiring the functional relationship specifically comprises: performing simulation on the basis of the plurality of different preset blood boron concentrations and the preset treatment plan to obtain the dose rate corresponding to each of the preset blood boron concentrations, and performing fitting on the basis of the plurality of preset blood boron concentrations and the dose rate corresponding to each of the preset blood boron concentrations to obtain the functional relationship.

12. The working method according to claim 10, wherein acquiring the corrected irradiation time specifically comprises: acquiring, on the basis of the actual blood boron concentration and the functional relationship, an estimated dose rate corresponding to the actual blood boron concentration; and correcting preset irradiation time on the basis of the estimated dose rate to obtain the corrected irradiation time.
